# EUROPÄISCHE PATENTSCHRIFT

(11) **EP 2 442 708 B1**
(45) Veröffentlichungstag und Bekanntmachung des Hinweises auf die Patenterteilung: **20.04.2016**
(21) Anmeldenummer: 10722016.2
(22) Anmeldetag: 04.06.2010
(51) Int. Cl.: A61B 5/00, A61B 5/15

(54) **Stechsystem**
Piercing system
Système de piquage

(30) Priorität: 19.06.2009 EP 09008023
(43) Veröffentlichungstag der Anmeldung: 25.04.2012
(73) Patentinhaber: Roche Diagnostics GmbH, 68305 Mannheim (DE); F.Hoffmann-La Roche AG, 4070 Basel (CH)
(72) Erfinder: KONYA, Ahmet, 67165 Waldsee (DE)
(74) Vertreter: Mommer, Niels
(86) Internationale Anmeldenummer: PCT/EP2010/003392
(87) Internationale Veröffentlichungsnummer: WO 2010/145763

(56) Entgegenhaltungen:
- EP-A- 1 997 429
- EP-A- 2 047 798
- EP-A1- 2 050 392
- WO-A-2005/107596
- WO-A-2008/083844

## Beschreibung

Die Erfindung geht aus von einem Stechsystem mit den im Oberbegriff des Anspruchs 1 angegebenen Merkmalen.

Aus der WO 2008/083844 A1 ist ein derartiges Stechsystem bekannt, bei dem auf einem Trägerband zwischen Stechelementen jeweils ein Testelement angeordnet ist. Zum Erzeugen einer Stichwunde wird ein Trägerbandhalter zusammen mit einem ein Stechelement tragenden Trägerbandabschnitt in Stichrichtung bewegt. Zur Probenaufnahme wird das Trägerband weitertransportiert und anschließend der Trägerbandhalter erneut in Stichrichtung bewegt und so ein Testelement an eine zuvor mit einem Stechelement erzeugte Stichwunde herangeführt. Nachteilig hieran ist, dass für eine zuverlässige Probenaufnahme das Austreten einer relativ großen Probenmenge aus der Stichwunde erforderlich ist.

Aufgabe der Erfindung ist es, einen Weg aufzuzeigen, wie der Benutzerkomfort eines Stechsystems verbessert werden kann.

Diese Aufgabe wird durch ein Stechsystem mit den im Anspruch 1 angegebenen Merkmalen sowie durch ein Verfahren mit den im Anspruch 15 angegebenen Merkmalen gelöst. Vorteilhafte Weiterbildungen der Erfindung sind Gegenstand von Unteransprüchen.

Bei einem erfindungsgemäßen Stechsystem wird ein Abschnitt des Trägerbandes mit einem unbenutzten Testelement an einem im Trägerbandhalter oder in Bandtransportrichtung hinter dem Trägerbandhalter gehaltenen Trägerbandabschnitt vorbeigeführt. Zur Probenübergabe von einem Stechelement auf ein Testelement werden diese beiden Trägerbandabschnitte gegeneinander gedrückt.

Bei einem Einstich bleibt Körperflüssigkeit an einem Stechelement haften, die als Probe zur Bestimmung einer Analytkonzentration, beispielsweise der Glucosekonzentration, verwendet werden kann. Um die auf diese Weise gewonnene Körperflüssigkeitsmenge zu erhöhen, kann das Stechelement eine Probenaufnahmeeinrichtung, beispielsweise Vertiefungen, Durchbrüche oder einen Kapillarkanal in Form einer Rille oder eines Spalts, aufweisen.

Durch eine Probenübergabe von dem Stechelement zu einem Testelement kann vorteilhaft in kürzerer Zeit eine Analytkonzentration bestimmt werden, da mit einer einzigen Stichbewegung des Trägerbandhalters sowohl eine Stichwunde erzeugt als auch eine Körperflüssigkeitsprobe aufgenommen wird. Da bei einem erfindungsgemäßen Stechsystem kein selbstständiges Austreten von Körperflüssigkeit aus der Stichwunde erforderlich ist, kann zudem die Stichtiefe reduziert und damit eine schmerzärmere Probengewinnung realisiert werden.

Das eine Probe aufnehmende Testelement kann in Bandtransportrichtung vor oder hinter dem die Probe abgebenden Stechelement angeordnet sein. Bevorzugt ist das die Probe aufnehmende Testelement in Bandtransportrichtung aber hinter dem die Probe abgebenden Stechelement und damit hinter dem Trägerbandhalter angeordnet. Vorteilhaft kann auf diese Weise die Gefahr einer Verschmutzung des Trägerbandhalters durch Körperflüssigkeit reduziert werden.

Um einen Abschnitt des Trägerbandes mit einem unbenutzten Testelement an einem im Trägerbandhalter oder in Bandtransportrichtung hinter dem Trägerbandhalter gehaltenen Trägerbandabschnitt vorbeizuführen, kann eine Bandumlenkung, beispielsweise ein Stift oder eine Umlenkrolle, verwendet werden, die zwischen diesen beiden Trägerbandabschnitten angeordnet ist. Einer der beiden Trägerbandabschnitte führt dann in Bandtransportrichtung zu der Bandumlenkung hin der andere Trägerbandabschnitt von der Bandumlenkung weg. Die beiden Trägerbandabschnitte können auf diese Weise aneinander entlang, bevorzugt zueinander parallel, geführt werden.

Um die beiden vor der Aufwickelrolle angeordneten Trägerbandabschnitte gegeneinander zu drücken, können beide Trägerbandabschnitte aufeinander zu bewegt werden. Es genügt jedoch, einen der beiden Trägerbandabschnitt zu bewegen. Beispielsweise kann der das Stechelement tragende Trägerbandabschnitt zu dem das Testelement tragen Trägerbandabschnitt bewegt und gegen diesen gedrückt werden.

Vor einer Probenübergabe kann das Trägerband weitertransportiert werden, so dass sich das die Probe abgebende Stechelement bei der Probenübergabe nicht mehr in dem Trägerbandhalter befindet. Ein solcher Weitertransport ist aber nicht erforderlich. Bevorzugt erfolgt die Probenübergabe von einem in dem Trägerbandhalter angeordneten Stechelement auf ein Testelement, da auf diese Weise weniger Zeit zwischen einem Einstich und der Probenübergabe verstreicht, so dass mit einer Eintrocknung der Probe verbundene Probleme minimiert werden können.

Eine vorteilhafte Weiterbildung der Erfindung sieht vor, dass der Trägerbandhalter quer zur Stichrichtung beweglich ist. Durch eine entsprechende Bewegung kann zur Probenübergabe ein benutztes Stechelement gegen ein Testelement gedrückt werden, indem der Trägerbandhalter und damit der in ihm angeordnete Trägerbandabschnitt gegen den das unbenutzte Testfeld tragenden Trägerbandabschnitt gedrückt wird.

Die Stechelemente sind bei einem erfindungsgemäßen Stechsystem bevorzugt quer zur Längsrichtung orientiert, wie dies bei dem der WO 2008/083844 A1 bekannten System der Fall ist. Möglich ist es aber auch, die Stechelemente wie bei dem aus der US 2005/0245954 A1 bekannten System in Längsrichtung des Trägerbandes zu orientieren.

Ein erfindungsgemäßes Stechsystem wird bevorzugt von einem Stechgerät und einer in das Stechgerät einsetzbaren Bandkassette gebildet. Eine Bandkassette mit einer erfindungsgemäßen Bandführung ist Gegenstand des Anspruchs 14. Bevorzugt ist der Trägerbandhalter Teil der Bandkassette. In diesem Fall kann der Trägerbandhalter beim Einlegen der Bandkassette in ein Stechgerät an dessen Stechantrieb ankoppeln. Möglich ist es auch, dass der Trägerbandhalter Teil des Stechgeräts ist und beim Einlegen der Bandkassette in das Stechgerät einen Trägerbandabschnitt aufnimmt. Bevorzugt enthält die Bandkassette eine Vorratsrolle, auf der das unbenutzte Trägerband aufgewickelt ist. Das unbenutzte Trägerband kann in der Bandkassette aber auch zu einem Stapel gefaltet sein. Prinzipiell ist es aber auch möglich, ein erfindungsgemäßes Stechsystem als Wegwerfgerät zu realisieren, bei dem ein Austausch des Trägerbandes nicht vorgesehen ist und das bestimmungsgemäß entsorgt wird, sobald alle auf dem Trägerband angeordneten Stech- und Testelemente aufgebraucht sind.

Weitere Einzelheiten und Vorteile einer erfindungsgemäßen Stechsystems sowie eines erfindungsgemäßen Verfahrens zur Übergabe einer Körperflüssigkeitsprobe von einem auf einem Trägerband angeordneten Stechelement auf ein auf dem Trägerband angeordnetes Testelement werden an Ausführungsbeispielen unter Bezugnahme auf die beigefügten Zeichnungen erläutert. Gleiche und einander entsprechende Komponenten sind in den Zeichnungen mit übereinstimmenden Bezugszahlen bezeichnet. Es zeigen:
- Figur 1: eine schematische Darstellung eines erfindungsgemäßen Stechsystems;
- Figur 2: eine schematische Darstellung eines weiteren Ausführungsbeispiels; und
- Figur 3: eine Seitenansicht zu Figur 2.

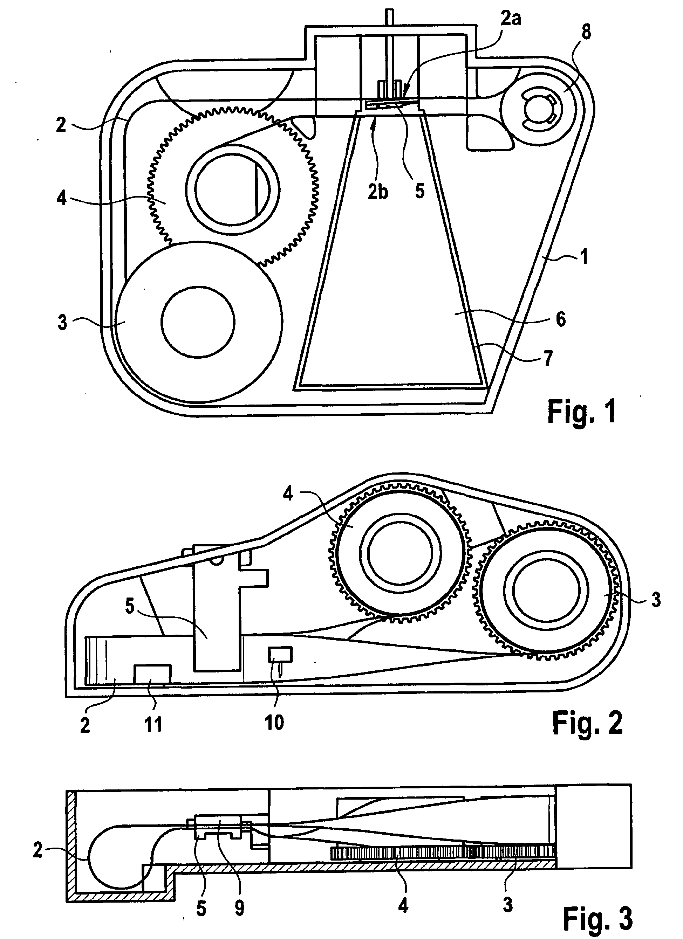

Figur 1 zeigt in einer schematischen Darstellung ein Ausführungsbeispiel eines Stechsystems bei geöffnetem Gerätegehäuse 1. In dem Gerätegehäuse 1 ist ein Trägerband 2 angeordnet, das Stechelemente und zwischen den Stechelementen angeordnete Testelemente zur Untersuchung einer durch einen Stich gewonnenen Körperflüssigkeitsprobe trägt. Das Trägerband 2 ist auf einer Vorratsrolle 3 aufgewickelt. Zum Gebrauch der Stechelemente und der Testelemente wird das Trägerband 2 schrittweise von der Vorratsrolle 3 abgewickelt und auf eine Aufwickelrolle 4 aufgewickelt, die auf diese Weise eine Bandtransporteinrichtung bildet.

Das Trägerband 1 wird von der Vorratsrolle 3 zu einem Trägerbandhalter 5 geführt, der mit einem in Figur 1 nicht dargestellten Stechantrieb gekoppelt ist und einen Abschnitt 2a des Trägerbandes 1 hält. Bei einem Stich bewegt sich der Trägerbandhalter 5 zusammen mit einem Stechelement, das der gehaltene Trägerbandabschnitt 2a trägt, in Stichrichtung. Bei dem dargestellten Ausführungsbeispiel sind die Stechelemente quer zur Längsrichtung des Trägerbandes orientiert, so dass die Stichrichtung senkrecht zur Zeichenebene der Figur 1 ist.

Die Analytkonzentration einer durch einen Stich gewonnen Körperflüssigkeitsprobe kann mit einer Messeinrichtung 6 gemessen werden. Die Messeinrichtung 6 wirkt dabei mit den Testelementen zusammen, die bevorzugt Nachweisreagenzien, beispielsweise für eine elektro-chemische oder photometrische Konzentrationsbestimmung enthalten. An sich kann die Messeinrichtung 6 aber auch für einen reagenzfreie Konzentrationsbestimmung, beispielsweise durch spektroskopische Analyse, vorgesehen sei. Bei dem dargestellten Ausführungsbeispiel ist die Messeinrichtung 6 für eine photometrische Konzentrationsbestimmung ausgebildet. Nachweisreagenzien der Testelemente bewirken hierfür eine konzentrationsabhängige Verfärbung, deren Intensität mit der Messeinrichtung 6 gemessen wird. Zur Minimierung von Streulicht hat die Messeinrichtung 6 ein Gehäuse 7, das von dem Gerätegehäuse 1 umgeben ist.

Bei einem Einstich in ein an eine Öffnung des Gerätegehäuses 1 angelegtes Körperteil eines Patienten, beispielsweise in einen Finger, bleibt Körperflüssigkeit an dem verwendeten Stechelement haften. Damit die so gewonnene Körperflüssigkeitsprobe untersucht werden kann, muss diese auf ein Testelement übertragen werden.

Bei dem dargestellten Ausführungsbeispiel werden zu diesem Zweck der Trägerbandabschnitt 2b mit einem unbenutzten Testelement und ein Trägerbandabschnitt 2a mit einem benutzen Stechelement gegeneinander gedrückt. Auf diese Weise wird ein Stechelement, an dem Körperflüssigkeit haftet, mit einem Testelement in Kontakt gebracht und so Körperflüssigkeit auf das Testelement übertragen.

Der Trägerbandabschnitt 2a mit dem die Probe abgegebenen Stechelement befindet sich bei dem gezeigten Ausführungsbeispiel in dem Trägerbandhalter 5. Der Trägerbandhalter 5 ist quer zur Stichrichtung und quer zur Längsrichtung des in ihm gehaltenen Trägerbandabschnitts 2a beweglich. Der Trägerbandhalter 5 kann deshalb zur Probenübergabe gegen einen an ihm vorbei geführten Trägerbandabschnitt 2b gedrückt werden. Bei dem dargestellten Ausführungsbeispiel wird der Trägerbandhalter 5 dabei auch gegen die Messeinrichtung 6, genauer gesagt gegen deren Gehäuse 7 gedrückt. Auf diese Weise kann ein Testfeld während einer Messung gegen die Messeinrichtung 6 gepresst werden, so dass definierte Bedingungen für eine präzise Messung vorliegen. Bevorzugt hat der Trägerbandhalter 5 auf einer Seite, die dem Trägerbandabschnitt 2b mit dem die Probe aufnehmenden Testfeld gegenüberliegt eine Aussparung. Vorteilhaft können so die beiden Trägerbandabschnitte 2a, 2b bei einer Probenübergabe flächig aneinander anliegen. Es genügt jedoch, wenn der Trägerbandhalter 5 das Trägerband 2 nur auf einem Teil seiner Breite hält und einen oberen Bereich mit den Spitzen der Stechelemente freilässt, so dass zur Probenübergabe die beiden Trägerbandabschnitte 2a, 2b mit ihrem oberen Bereich aneinander anliegen.

Prinzipiell kann das eine Probe aufnehmende Testelement in Bandtransportrichtung vor oder hinter dem Trägerbandhalter 5 angeordnet sein. Bei dem dargestellten Ausführungsbeispiel ist das eine Probe aufnehmende Testelement in Bandtransportrichtung hinter dem Trägerbandhalter 5 angeordnet. Vorteilhaft wird auf diese Weise die Gefahr einer Verschmutzung des Trägerbandhalters 5 durch Körperflüssigkeit vermieden.

Bei dem dargestellten Ausführungsbeispiel ist das Trägerband 2 von dem Trägerbandhalter 5 aus zu einer Umlenkrolle 8 und von dort an dem Trägerbandhalter 5 vorbei zu der Aufwickeleinrichtung 4 geführt. Auf diese Weise bildet das Trägerband 2 eine Schlaufe mit zwei aneinander entlang verlaufenden Trägerbandabschnitten, nämlich dem in dem Trägerbandhalter 5 gehaltenen Trägerbandabschnitt 2a und einem zwischen dem Trägerbandhalter 5 und der Messeinrichtung 6 verlaufenden Trägerbandabschnitt 2b. Zur Übergabe einer Probe von einem Stechelement auf ein Testfeld werden die beiden Trägerbandabschnitte 2a, 2b, die bevorzugt näherungsweise parallel verlaufen, zusammengedrückt, so dass das Stechelement das Testelement kontaktiert.

Die Länge der Bandschlaufe ist dabei passend zu dem Abstand von einem Stechelement zu dem ihm zugeordneten Testelement bemessen. Dabei ist es aber nicht erforderlich, dass die Länge der durch die Umlenkrolle 8 gebildeten Bandschlaufe dem Abstand zwischen einem Stechelement und dem benachbarten Testelement entspricht. Es ist nämlich auch möglich, dass eine Probenübergabe von einem Stechelement nicht zu einem benachbarten Testelement, sondern zu einem weiter entfernten angeordneten Stechelement, beispielsweise dem übernächsten oder überübernächsten Stechelement erfolgt.

Der Trägerbandhalter 5 kann im einfachsten Fall als ein Schlitz mit einer stets gleich bleibenden Breite ausgebildet sein. Bevorzugt weist der Trägerbandhalter 5 aber zwei gegeneinander bewegliche Teile auf, zwischen denen das Trägerband 2 hindurchgeführt ist. Auf diese Weise kann der Trägerbandhalter 5 das Trägerband 2 bei einem Stich zwischen den beiden gegeneinander beweglichen Teilen klemmend halten. Zum Bandtransport kann das Trägerband 2 freigegeben werden, also der Abstand zwischen den beiden gegeneinander beweglichen Teilen vergrößert werden, so dass mit geringer Bandreibung durch Aufwickeln der Aufwickelrolle 4 das nächste Stechelement in seine Gebrauchsposition in dem Trägerbandhalter 5 gebracht werden kann. Beispielsweise können die beiden gegeneinander beweglichen Teile des Trägerbandhalters 5 schwenkbar beweglich sein. Nach diesem Prinzip sind auch die zusammen wirkenden Schenkel einer Zange beweglich, um etwas klemmend zu halten.

Bevorzugt erfolgt zwischen einem Stich und einer Probenübergabe auf ein Testelement kein Bandtransport, d. h. die Aufwickelrolle 4 wird zwischen einem Stich und einer Probenübergabe nicht bewegt. Die Gebrauchsposition eines Stechelements in dem Trägerbandhalter 5, die es bei einem Stich inne hat, wird demnach bevorzugt auch zur Probenübergabe genutzt.

Die Bewegung des Trägerbandhalters 5 zum Zusammenpressen der beiden Trägerbandabschnitte 2a, 2b bei einer Probenübergabe wird bevorzugt von dem Stechantrieb im Anschluss an eine Stichbewegung selbsttätig bewirkt. Beispielsweise kann der Stechantrieb hierfür eine geeignete Kulissensteuerung aufweisen. Es ist nämlich vorteilhaft, wenn eine Probe sobald wie möglich von einem Stechelement an ein Testelement übergeben wird. Insbesondere bei trockener Umgebungsluft besteht nämlich die Gefahr, dass Körperflüssigkeit verdunstet und eine Probe am Stechelement eintrocknet, so dass eine Probenübergabe nur in einem kurzen Zeitfenster möglich ist.

Die Figuren 2 und 3 zeigen ein weiteres Ausführungsbeispiels bei geöffnetem Gerätegehäuse 1 in einer schematischen Draufsicht und eine dazugehörenden Seitenansicht, in welcher auch der mit dem Trägerbandhalter 5 gekoppelte Stechantrieb 9 dargestellt ist. Der Unterschied zu dem vorstehend beschriebenen Ausführungsbeispiel besteht dabei im Wesentlichen darin, dass das Trägerband 2 auf seinem Weg von der Vorratsrolle 3 zu dem Trägerbandhalter 5 eine Vierteldrehung durchführt. Die geometrische Drehachse der Vorratsrolle 3 ist deshalb nicht wie bei dem vorstehend beschriebenen Ausführungsbeispiel parallel zur Stichrichtung, sondern quer dazu orientiert.

Zwischen der Messeinrichtung 6 und der Aufwickelrolle 4 ist das Trägerband 2 um eine weitere Vierteldrehung verdrillt. Die geometrische Drehachse der Aufwickelrolle 4 ist deshalb parallel zur geometrischen Drehachse der Vorratsrolle 3 orientiert. Die zweite Vierteldrehung des Trägerbandes 2 kann die vorhergehende Vierteldrehung rückgängig machen oder sich dazu addieren, so dass das Trägerband 2 insgesamt um eine halbe Drehung verdrillt ist.

In Figur 2 sind die in Figur 1 nicht dargestellten Stechelemente 10 und Testelemente 11 auf dem Trägerband 2 zu erkennen. Die Stechelemente 10 können aus Metall, insbesondre Stahl, hergestellt werde, wobei jedoch auch andere Materialien wie Kunststoff oder Keramik verwendet werden können. Die Stechelemente 10 haben eine Probenaufnahmeeinrichtung, die beispielsweise als ein Kapillarkanal oder Vertiefungen bzw. Durchbrüche, in denen Körperflüssigkeit bei einem Stich haften bleibt, ausgebildet sein kann. Ein Kapillarkanal ist ein Kanal, in dem Körperflüssigkeit bei einem Stich durch Kapillarkräfte gehalten wird. Ein solcher Kanal kann als eine Rinne ausgebildet sein oder ein Schlitz sein, der zur Ober- und Unterseite des Stechelements 10 hin offen ist.

Die Testelemente 11 können als Testfelder auf dem Trägerband 2 vorgesehen sein. Die Testelemente 11 haben eine saugfähige Oberfläche, um eine Probenaufnahme von einem Stechelement 10 zu erleichtern. Die saugfähige Oberfläche kann beispielsweise durch Auftragen einer Paste oder durch ein Vlies gebildet werden und Nachweisreagenzien enthalten. Für eine photometrische Konzentrationsbestimmung kann es vorteilhaft sein, ein transparentes Trägerband 2 zu verwenden, so dass Messlicht durch das Trägerband 2 hindurch gelangen kann. Möglich ist es auch, Testelemente auf passende Fenster des Trägerbandes 2 aufzukleben.

### Bezugszahlen

- 1: Gerätegehäuse
- 2: Trägerband
- 2a: Trägerbandabschnitt
- 2b: Trägerbandabschnitt
- 3: Vorratsrolle
- 4: Aufwickelrolle
- 5: Trägerbandhalter
- 6: Messeinrichtung
- 7: Gehäuse
- 8: Umlenkrolle
- 9: Stechantrieb
- 10: Stechelement
- 11: Testelement

## Patentansprüche

1. Stechsystem mit
einem Trägerband (2), das Stechelemente (10) und zwischen den Stechelementen (10) angeordnete Testelemente (11) zur Untersuchung einer durch einen Stich gewonnenen Körperflüssigkeitsprobe trägt,
einem Stechantrieb (9), um Stechelemente (10) in eine Stichbewegung zu versetzen,
einen mit dem Stechantrieb (9) gekoppelten Trägerbandhalter (5), der einen Abschnitt des Trägerbandes (2) hält und sich bei einem Stich zusammen mit diesem Abschnitt (2a) und einem von dem Abschnitt (2a) getragenen Stechelement (10) in Stichrichtung bewegt,
einer Aufwickelrolle (4), um das Trägerband (2) durch Aufwickeln in einer Bandtransportrichtung zu bewegen,
einer Messeinrichtung (6) zur Messung einer Analytkonzentration einer von einem Testelement aufgenommenen Körperflüssigkeitsprobe,
**dadurch gekennzeichnet, dass**
ein Abschnitt (2b) des Trägerbandes (2) mit einem unbenutzten Testelement (11) an einem Trägerbandabschnitt (2a) vorbeigeführt ist, der im Trägerbandhalter (5) oder in Bandtransportrichtung hinter dem Trägerbandhalter (5) angeordnet ist, und zur Probenübergabe von einem Stechelement (10) auf ein Testelement (11) diese beiden Trägerbandabschnitte (2a, 2b) gegeneinander gedrückt werden.

2. Stechsystem nach Anspruch 1, **dadurch gekennzeichnet, dass** die beiden Trägerbandabschnitte (2a, 2b) während einer Messung gegeneinander gedrückt werden.

3. Stechsystem nach einem der vorstehenden Ansprüche, **dadurch gekennzeichnet, dass** das Trägerband (2) zwischen den beiden Trägerbandabschnitten(2a, 2b), die zur Probenübergabe gegeneinander gedrückt werden, eine Schlaufe bildet.

4. Stechsystem nach einem der vorstehenden Ansprüche, dadurch **gekennzeich-net,** dass das Trägerband (2) zwischen den beiden Trägerbandabschnitten (2a, 2b), die zur Probenübergabe gegeneinander gedrückt werden, um eine Umlenkrolle (8) geführt ist.

5. Stechsystem nach einem der vorstehenden Ansprüche, dadurch **gekennzeich-net,** dass die Probenübergabe von einem Stechelement (10) zu einem Testelement (11) erfolgt, das in Bandtransportrichtung vor dem Stechelement (10) auf dem Trägerband (2) angeordnet ist.

6. Stechsystem nach einem der vorstehenden Ansprüche, dadurch **gekennzeich-net,** dass zur Probenübergabe ein das Stechelement (10) tragender Trägerbandabschnitt (2a) zu einem ein Testelement (11) tragenden Bandabschnitt (2b) hin bewegt wird.

7. Stechsystem nach einem der vorstehenden Ansprüche, dadurch **gekennzeich-**net, dass die Probenübergabe von einem in dem Trägerbandhalter (5) angeordneten Stechelement (10) auf ein Testelement (11) erfolgt.

8. Stechsystem nach einem der vorstehenden Ansprüche, dadurch **gekennzeich-net,** dass der Trägerbandhalter (5) quer zur Stichrichtung beweglich ist, um zur Probenübergabe ein benutztes Stechelement (10) gegen ein Testelement (11) zu drücken.

9. Stechsystem nach einem der vorstehenden Ansprüche, dadurch **gekennzeich-net,** dass die Stechelemente (10) quer zur Längsrichtung des Trägerbands (2) orientiert sind.

10. Stechsystem nach einem der vorstehenden Ansprüche, **dadurch gekennzeichnet, dass** der Trägerbandhalter (5) zwei gegeneinander bewegliche Teile aufweist, zwischen denen das Trägerband (2) hindurchgeführt ist.

11. Stechsystem nach Anspruch 10, **dadurch gekennzeichnet, dass** der Trägerbandhalter (5) das Trägerband (2) bei einem Stich zwischen den beiden gegeneinander beweglichen Teilen klemmt.

12. Stechsystem nach einem der vorstehenden Ansprüche, dadurch **gekennzeich-net,** dass das Trägerband (2) bei einer Messung gegen die Messeinrichtung (6) gedrückt wird.

13. Stechsystem nach Anspruch 12, **dadurch gekennzeichnet, dass** das Trägerband (2) von dem Trägerbandhalter (5) gegen das Gehäuse (7) der Messeinrichtung gedrückt wird.

14. Bandkassette für ein Stechgerät, das zusammen mit der in das Stechgerät einsetzbaren Bandkassette ein Stechsystem bildet, wobei die Bandkassette ein Trägerband (2), das Stechelemente (10) und zwischen den Stechelementen (10) angeordnete Testelemente (11) zur Untersuchung einer durch einen Stich gewonnenen Körperflüssigkeitsprobe trägt, und eine Aufwickelrolle (4) zum Aufwickeln des Trägerbandes (2) enthält, **dadurch gekennzeichnet, dass** zwei Trägerbandabschnitte (2a, 2b) aneinander vorbeigeführt sind, so dass zur Übergabe einer Probe von einem Stechelement (10) auf ein Testelement (11) diese beiden Trägerbandabschnitte (2a, 2b) gegeneinander gedrückt werden können.

15. Verfahren zur Übergabe einer Körperflüssigkeitsprobe von einem auf einem Trägerband (2) angeordneten Stechelement (10) auf ein auf dem Trägerband (2) angeordnetes Testelement (11), **dadurch gekennzeichnet, dass** das Trägerband (2) um eine Bandumlenkung (8) geführt ist, die zwischen einem das Stechelement (10) tragenden Trägerbandabschnitt (2a) und einem das Testelement (11) tragenden Trägerbandabschnitt (2b) angeordnet ist, und die beiden Trägerbandabschnitt (2a, 2b) zur Probenübergabe zusammengedrückt werden, so dass das Stechelement (10) das Testelement (11) kontaktiert.

## Claims

1. A piercing system comprising
a carrier tape (2), which carries piercing elements (10) and test elements (11) for testing a sample of bodily fluid obtained by pricking, the test elements (11) being disposed between the piercing elements (10),
a piercing drive (9) for causing a pricking movement of piercing elements (10),
a carrier tape holder (5) coupled to the piercing drive (9) and holding a segment of the carrier tape (2), the carrier tape holder (5) moving, together with this segment (2a) and a piercing element (10) carried by the segment (2a), in the pricking direction during pricking,
a wind-up reel (4), for moving the carrier tape (2) in a tape transport direction by winding,
a measuring device (6) for measuring an analyte concentration in a sample of bodily fluid collected by a test element,
**characterized in that**
a segment (2b) of the carrier tape (2) having an unused test element (11) is guided past a carrier tape segment (2a), which is disposed in the carrier tape holder (5) or behind the carrier tape holder (5) in the tape transport direction, and said two carrier tape segments (2a, 2b) are pressed against one another for transferring a sample from a piercing element (10) to a test element (11).

2. The piercing system according to claim 1, **characterized in that** the two carrier tape segments (2a, 2b) are pressed against one another during a measurement.

3. The piercing system according to any one of the preceding claims, **characterized in that** the carrier tape (2) between the two carrier tape segments (2a, 2b) that are pressed against one another for transferring a sample forms a loop.

4. The piercing system according to any one of the preceding claims, **characterized in that** the carrier tape (2) between the two carrier tape segments (2a, 2b) that are pressed against one another for transferring a sample is guided around a deflecting roller (8).

5. The piercing system according to any one of the preceding claims, **characterized in that** the sample is transferred from a piercing element (10) to a test element (11), which is disposed ahead of the piercing element (10) on the carrier tape (2) in the tape transport direction.

6. The piercing system according to any one of the preceding claims, **characterized in that** for transferring a sample, a carrier tape segment (2a) that carries the piercing element (10) is moved toward a tape segment (2b) that carries a test element (11).

7. The piercing system according to any one of the preceding claims, **characterized in that** the sample is transferred from a piercing element (10) disposed in the carrier tape holder (5) to a test element (11).

8. The piercing system according to any one of the preceding claims, **characterized in that** the carrier tape holder (5) is movable transversely to the pricking direction, to allow a used piercing element (10) to be pressed against a test element (11) for transferring a sample.

9. The piercing system according to any one of the preceding claims, **characterized in that** the piercing elements (10) are oriented transversely to the longitudinal direction of the carrier tape (2).

10. The piercing system according to any one of the preceding claims, **characterized in that** the carrier tape holder (5) has two parts that are movable relative to one another, between which the carrier tape (2) passes.

11. The piercing system according to claim 10, **characterized in that** during a pricking, the carrier tape holder (5) clamps the carrier tape (2) between the two parts that are movable relative to one another.

12. The piercing system according to any one of the preceding claims, **characterized in that** the carrier tape (2) is pressed against the measuring device (6) during a measurement.

13. The piercing system according to claim 12, **characterized in that** the carrier tape (2) is pressed by the carrier tape holder (5) against the housing (7) of the measuring device.

14. A tape cartridge for a piercing system according to any one of the preceding claims, the tape cartridge containing a carrier tape (2), which carries piercing elements (10) and test elements (11) disposed between the piercing elements (10), for testing a sample of bodily fluid obtained by a prick, and a wind-up reel (4) for winding up the carrier tape (2), **characterized in that** two carrier tape segments (2a, 2b) are guided past one another, so that, to transfer a sample from a piercing element (10) to a test element (11), these two carrier tape segments (2a, 2b) can be pressed against one another.

15. A method for transferring a sample of bodily fluid from a piercing element (10) disposed on a carrier tape (2) to a test element (11) disposed on the carrier tape (2), **characterized in that** the carrier tape (2) is guided around a tape deflection, which is disposed between a carrier tape segment (2a) that carries the piercing element (10) and a carrier tape segment (2b) that carries the test element (11), and the two carrier tape segments (2a, 2b) are pressed together for transferring a sample, such that the piercing element (10) comes into contact with the test element (11).

## Revendications

1. Système de piquage avec
une bande support (2), qui porte des éléments de piquage (10) et des éléments de test (11) agencés entre les éléments de piquage (10) en vue d'un examen d'un échantillon de liquide corporel obtenu grâce à une piqure,
un entrainement de piquage (9), pour déplacer des éléments de piquage (10) avec un mouvement de piquage,
un porte-bande support (5), couplé à l'entrainement de piquage (9), qui tient une section de la bande support (2) et, lors d'un piquage, se déplace dans une direction de piquage en même temps que ladite section (2a) et un élément de piquage (10) porté par la section (2a),
un cylindre d'enroulement (4), pour déplacer la bande support (2) par enroulement dans une direction de transport de bande,
un appareil de mesure (6) pour une mesure d'une concentration en analyte d'un échantillon de liquide corporel accueilli par un élément de test,
**caractérisé en ce que**
une section (2b) de la bande support (2) avec un élément de test (11) inutilisé est acheminée au niveau d'une section de bande support (2a) qui est agencée dans le porte-bande support (5) ou, dans une direction de transport de bande, derrière le porte-bande support (5), et lesdites deux sections de bande support (2a, 2b) sont pressées l'une contre l'autre en vue d'un transfert d'échantillon d'un élément de piquage (10) vers un élément de test (11).

2. Système de piquage selon la revendication 1, **caractérisé en ce que** les deux sections de bande support (2a, 2b) sont pressées l'une contre l'autre pendant une mesure.

3. Système de piquage selon l'une quelconque des revendications précédentes, **caractérisé en ce que** la bande support (2) forme une boucle entre les deux sections de bande support (2a, 2b) qui sont pressées l'une contre l'autre en vue d'un transfert d'échantillon.

4. Système de piquage selon l'une quelconque des revendications précédentes, **caractérisé en ce que** la bande support (2) est guidée autour d'un cylindre de renvoi (8) entre les deux sections de bande support (2a, 2b) qui sont pressées l'une contre l'autre en vue d'un transfert d'échantillon.

5. Système de piquage selon l'une quelconque des revendications précédentes, **caractérisé en ce que** le transfert d'échantillon a lieu d'un élément de piquage (10) vers un élément de test (11) qui, dans une direction de transport de bande, est agencé sur la bande support (2) avant l'élément de piquage (10).

6. Système de piquage selon l'une quelconque des revendications précédentes, **caractérisé en ce qu'**une section de bande support (2a) portant l'élément de piquage (10) est déplacée vers une section de bande (2b) portant un élément de test (11) en vue d'un transfert d'échantillon.

7. Système de piquage selon l'une quelconque des revendications précédentes, **caractérisé en ce que** le transfert d'échantillon a lieu d'un élément de piquage (10) agencé dans le porte-bande support (5) vers un élément de test (11).

8. Système de piquage selon l'une quelconque des revendications précédentes, **caractérisé en ce que** le porte-bande support (5) est mobile perpendiculairement à une direction de piquage, afin de presser un élément de piquage (10) utilisé contre un élément de test (11) en vue d'un transfert d'échantillon.

9. Système de piquage selon l'une quelconque des revendications précédentes, **caractérisé en ce que** les éléments de piquage (10) sont orientés perpendiculairement à une direction longitudinale de la bande support (2).

10. Système de piquage selon l'une quelconque des revendications précédentes, **caractérisé en ce que** le porte-bande support (5) présente deux parties mobiles l'une contre l'autre, entre lesquelles on fait passer la bande support (2).

11. Système de piquage selon la revendication 10, **caractérisé en ce que** le porte-bande support (5), lors d'un piquage, pince la bande support (2) entre les deux parties mobiles l'une contre l'autre.

12. Système de piquage selon l'une quelconque des revendications précédentes, **caractérisé en ce que** la bande support (2) est pressée contre l'appareil de mesure (6) lors d'une mesure.

13. Système de piquage selon la revendication 12, **caractérisé en ce que** la bande support (12) est pressée contre le boîtier (7) de l'appareil de mesure par le porte-bande support (5).

14. Cassette à bande pour un appareil de piquage, qui forme, en commun avec la cassette à bande insérable dans l'appareil de piquage, un système de piquage, ladite cassette à bande contenant une bande support (2) qui porte des éléments de piquage (10) et des éléments de test (11) agencés entre les éléments de piquage (10) en vue d'un examen d'un échantillon de fluide corporel obtenu grâce à une piqure, et contenant un cylindre d'enroulement (4) en vue d'un enroulement de la bande support (2), **caractérisé en ce que** deux sections de bande support (2a, 2b) défilent l'une au niveau de l'autre, de telle manière que lesdites deux sections de bande support (2a, 2b) peuvent être pressées l'une contre l'autre en vue d'un transfert d'un échantillon d'un élément de piquage (10) vers un élément de test (11).

15. Procédé de transfert d'un échantillon de fluide corporel d'un élément de piquage (10) agencé sur une bande support (2) vers un élément de test (11) agencé sur la bande support (2), **caractérisé en ce que** la bande support (2) est guidée autour d'un renvoi de bande (8) qui est agencé entre une section de bande support (2a) portant l'élément de piquage (10) et une section de bande support (2b) portant l'élément de test (11), et les deux sections de bande support (2a, 2b) sont pressées ensemble en vue d'un transfert d'échantillon de telle manière que l'élément de piquage (10) vient en contact avec l'élément de test (11).
